# EUROPÄISCHE PATENTSCHRIFT

(11) **EP 1 157 015 B1**
(45) Veröffentlichungstag und Bekanntmachung des Hinweises auf die Patenterteilung: **11.05.2005**
(21) Anmeldenummer: 00910642.8
(22) Anmeldetag: 05.02.2000
(51) Int. Cl.: C07D 277/60, C07D 277/84, A61K 31/425, C07D 417/04

(54) **VERWENDUNG VON POLYCYCLISCHEN 2-AMINO-THIAZOL SYSTEMEN ZUR HERSTELLUNG VON MEDIKAMENTEN ZUR BEHANDLUNG VON FETTSTOFFWECHSELSTÖRUNGEN, OBESITAS UND TYP II DIABETES**
USE OF POLYCYCLIC 2-AMINO-THIAZOLE SYSTEMS FOR THE MANUFACTURE OF MEDICAMENTS FOR THE TREATMENT OF LIPOMETABOLIC DISORDERS, OBESITAS AND TYP II DIABETES
UTILISATION DE SYSTEMES 2-AMINO-THIAZOLE POLYCYQUES POUR LA PREPARATION DE MEDICAMENTS POUR LE TRAITEMENT DES TROUBLES DU LIPOMETABOLISME, OBESITAS ET DIABETES TYPE II

(30) Priorität: 26.02.1999 DE 19908538
(43) Veröffentlichungstag der Anmeldung: 28.11.2001
(73) Patentinhaber: Aventis Pharma Deutschland GmbH, 65929 Frankfurt am Main (DE)
(72) Erfinder: JÄHNE, Gerhard, D-65929 Frankfurt (DE); GEISEN, Karl, D-60318 Frankfurt (DE); LANG, Hans-Jochen, D-65719 Hofheim (DE); BICKEL, Martin, D-61348 Bad Homburg (DE)
(86) Internationale Anmeldenummer: PCT/EP2000/000924
(87) Internationale Veröffentlichungsnummer: WO 2000/051995

(56) Entgegenhaltungen:
- EP-A- 0 230 334
- EP-A- 0 432 040
- WO-A-98/13356
- WO-A-99/42455
- DE-A- 19 831 878
- US-A- 4 174 397
- CHEMICAL ABSTRACTS, vol. 120, no. 19, 9. Mai 1994 (1994-05-09) Columbus, Ohio, US; abstract no. 244848, PEESAPATI, V. ET AL: "Thiopheno[3,2][1]benzazepine, benzo[3,4]cyclohepta[2,1-b]thiophenes, thiazolo[5,4-d][1]benzazepine and benzo[3,4]cyclohepta[2,1- d]thiazoles" XP002141367 & ORG. PREP. PROCED. INT. (1993), 25(5), 602-6 , 1993,
- CHEMICAL ABSTRACTS, vol. 117, no. 11, 14. September 1992 (1992-09-14) Columbus, Ohio, US; abstract no. 111516, GUPTA, RAJIVE ET AL: "Synthesis and antiinflammatory activity of some substituted 2-amino-8H-indeno[1,2-d]thiazoles" XP002141368 & INDIAN J. PHARM. SCI. (1991), 53 245-8 , 1991,
- CHEMICAL ABSTRACTS, vol. 116, no. 15, 13. April 1992 (1992-04-13) Columbus, Ohio, US; abstract no. 151654, BERLIN, K. DARRELL ET AL: "Novel 2-amino-4-aryl-substituted and 2-amino-4,5-disubstituted thiazoles" XP002141369 & PROC. OKLA. ACAD. SCI. (1991), 71, 29-33 , 1991,
- CHEMICAL ABSTRACTS, vol. 112, no. 13, 26. März 1990 (1990-03-26) Columbus, Ohio, US; abstract no. 118405, SINGH, RAJESHWAR ET AL: "Synthesis and pharmacological evaluation of Mannich bases and other related compounds, derived from 2,3-dihydro-1H-inden-1-ones and 2,3,4,5-tetrahydrobenzocyclohepten-1H-one" XP002141370 & INDIAN J. CHEM., SECT. B (1989), 28B(6), 486-9 , 1989,
- CHEMICAL ABSTRACTS, vol. 86, no. 23, 6. Juni 1977 (1977-06-06) Columbus, Ohio, US; abstract no. 165780, RAMALINGAM, K. ET AL: "Synthesis and antimicrobial activity of azasteroid-type compounds and related systems. Effect of hydrophilic and lipophilic groups on activity" XP002141371 & J. MED. CHEM. (1977), 20(5), 664-9 , 1977,
- CHEMICAL ABSTRACTS, vol. 84, no. 11, 15. März 1976 (1976-03-15) Columbus, Ohio, US; abstract no. 69717, HASHEM, M. M. ET AL: "Novel pyrazolo, isoxazolo, and thiazolo steroidal systems and model analogs containing dimethoxylaryl (or dihydroxylaryl) groups and derivatives. Synthesis, spectral properties, and biological activity" XP002141372 & J. MED. CHEM. (1976), 19(2), 229-39 , 1976,
- CHEMICAL ABSTRACTS, vol. 77, no. 17, 23. Oktober 1972 (1972-10-23) Columbus, Ohio, US; abstract no. 113984, SHAKHNAZARYAN, G. M. ET AL: "Molecular rearrangements. X. Oxidation of dichlorovinyl compounds containing aromatic substituents by peracetic acid" XP002141373 & ARM. KHIM. ZH. (1972), 25(5), 415-20 , 1972,

## Beschreibung

Die Erfindung betrifft die Verwendung von polycyclischen 2-Amino-Thiazol Systemen sowie derer physiologisch verträglichen Salze zur Herstellung eines Medikaments zur Behandlung von Fettstoffwechselstörungen, Obesitas und Typ II Diabetes.

2-Amino-Thiazolsysteme sind in R. Gupta et al., Indian J. Pharm. Sci. 1991, 53, 245-248 als entzündungshemmende Substanzen beschrieben

WO 99/42455 offenbart polycyclische 2-Amino-Thiazol Systeme und deren Verwendung als antivirale Wirkstoffe.

Der Erfindung lag die Aufgabe zugrunde, Verbindungen zur Verfügung zu stellen, die eine therapeutisch verwertbare anorektische Wirkung entfalten.

Die Erfindung betrifft daher die Verwendung von Verbindungen der Formel I, 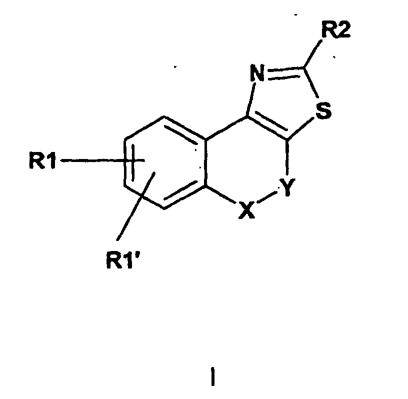 worin bedeuten
- Y: eine direkte Bindung;
- X: CH₂;
- R1: CF₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkyl-, Alkenyl- und Alkinylresten ein, mehrere, oder alle Wasserstoff(e) durch Fluor ersetzt ist;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
Phenyl, O-Phenyl, wobei der Phenylrest bis zu 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
- R1': H, CF₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkyl-, Alkenyl- und Alkinylresten ein, mehrere, oder alle Wasserstoff(e) durch Fluor ersetzt ist;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
Phenyl, O-Phenyl, wobei der Phenylrest bis zu 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
- R2: NH₂, NHR3, NR4R5;
- R3: (C₁-C₆)-Alkyl;
- R4: (C₁-C₆)-Alkyl;
- R5: (C₁-C₆)-Alkyl;
sowie derer physiologisch verträglichen Salze zur Herstellung eines Medikaments zur Behandlung von Fettstoffwechselstörungen, Obesitas und Typ II Diabetes.

Die Erfindung bezieht sich auf die Verwendung der Verbindungen der Formel I, in Form ihrer Racemate, racemischen Mischungen und reinen Enantiomere sowie auf ihre Diastereomere und Mischungen davon.
Die Alkyl-, Alkenyl- und Alkinylreste in den Substituenten R1, R1', R2, R3, R4 und R5 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isäthion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorid verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden; indem man eine Verbindung gemäß Formel (I) mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzeine Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formel I können gemäß folgendem Reaktionsschema hergestellt werden:

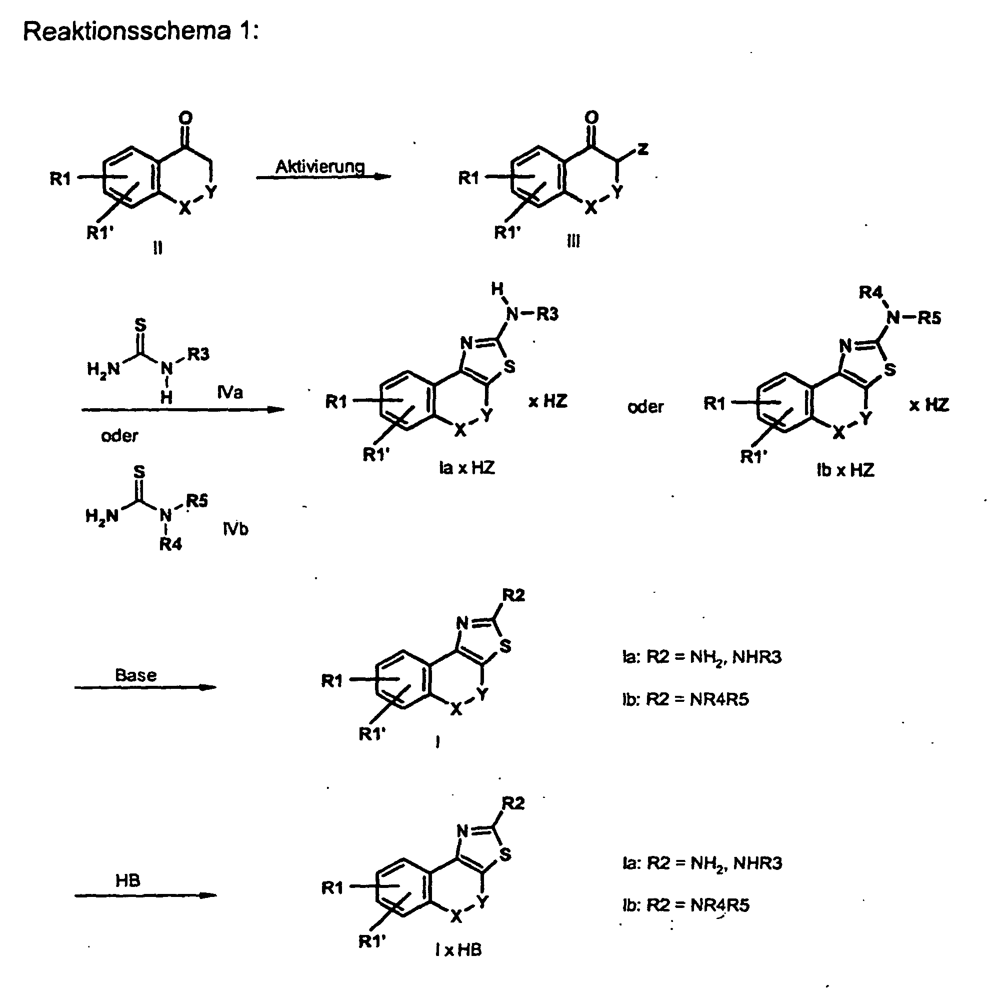

Bicyclische Ketone der allgemeinen Formel II, worin R1, R1', X und Y die angegebenen Bedeutungen besitzen, sind entweder kommerziell erhältlich oder nach literaturbekannten Methoden herstellbar.

Bicyclische Ketone der Formel II, worin R1 oder R1' Arylreste darstellen, können durch Pd(0)-katalysierte Addition von Boronsäureestem an Verbindungen der Formel II, worin R1 und/oder R1' Brom, Jod oder Trifluormethylsulfonyloxy darstellen, gewonnen werden (z.B.: N. Miyaura und A. Suzuki, Chem. Rev. 95, 2457-83 (1995) oder T. Oh-e, N. Miyaura und A. Suzuki, J. Org. Chem. 58, 2201-08 (1933).

Bicyclische Ketone der allgemeinen Formel II, worin R1 und/oder R1' Alkinylreste oder Alkenylreste darstellen, können z.B. mit Methoden wie sie bei K. Sonagashira et al., Tetrahedron Lett. 4467 (1975) und S. Takahashi et al., Synthesis 627 (1980) (palladiumkatalysierte Umsetzung von z. B. Trimethylsilylacetylen oder Alkinen) oder bei E. Negishi et al., J. Org. Chem. 62, 8957-60 (1997) (Alkinylzinkbromide) oder bei A. Hassner et al., J. Org. Chem. 49, 2546 (1984) (Trialkylzinnalkine, Trialkylzinnvinyl- oder allylverbindungen, 1-Alkenylborverbindungen oder Vinylverbindungen) beschrieben sind, hergestellt werden.

Die Aktivierung der bicyclischen Ketone der allgemeinen Formel II erfolgt am einfachsten durch Umsetzung mit Brom zum alpha-Bromketon der allgemeinen Formel III (Z = Br). Z in den aktivierten Verbindungen der allgemeinen Formel III kann jedoch auch vorteilhaft Cl, J. O-C(O)-C₆H₄-4-NO₂, O-SO₂-CH₃, O-SO₂-CF₃, O-SO₂-C₆H₄-4-CH₃ oder O-SO₂-C₆H₄ sein.
Verbindungen der allgemeinen Formel I x HZ werden gewonnen, indem man Thioharnstoffe der allgemeinen Formel IVa oder IVb, worin R2 = NH₂, NHR3 oder R2 = NR4R5 ist und die Reste R2, R3, R4 und R5 die angegebenen Bedeutungen besitzen, umsetzt. Dabei wird vorteilhaft so verfahren, daß man die Verbindungen III mit den Thiohamstoffen IVa oder IVb im molaren Verhältnis von 1:1 bis 1:1,5 umsetzt. Die Reaktion wird vorteilhaft in einem inerten Lösemittel, z.B. in polaren organischen Lösemitteln wie Dimethylformamid, Dimethylacetamid, N-Methyl-2-pyrrolidon, Dimethylsulfoxid, Dioxan, Tetrahydrofuran, Acetonitril, Nitromethan oder Diethylenglykoldimethylether durchgeführt. Als besonders vorteilhafte Lösemittel erweisen sich jedoch Essigsäuremethylester und: Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z.B. Gemische aus Methanol mit Benzol, Ethanol mit Toluol, Methanol mit Diethylether oder mit tert.Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen. Grundsätzlich können die Reaktionspartner auch ohne Lösemittel umgesetzt werden, insbesondere dann, wenn das jeweilige Thioamid einen tiefen Schmelzpunkt hat. Die Reaktion verläuft nur wenig exotherm und kann zwischen - 10°C und 150°C, bevorzugt zwischen 50°C und 100°C, durchgeführt werden. Als besonders günstig erweist sich in der Regel ein Temperaturbereich zwischen 50°C und 80°C.

Die Reaktionsdauer ist weitgehend von der Reaktionstemperatur abhängig und liegt zwischen 2 Minuten und 3 Tagen bei höheren bzw. niedrigeren Temperaturen. Im günstigen Temperaturbereich liegt die Reaktionsdauer im allgemeinen zwischen 5 Minuten und 48 Stunden.
Die erhaltenen Salze der Verbindungen der allgemeinen Formel la x HZ und Ib x HZ lassen sich mit organischen oder anorganischen Basen in die freien basischen Verbindungen der Formel I (Ia: R2 = NH₂, NHR3; Ib: R2 = NR4R5) überführen. Die Verbindungen der allgemeinen Formel I lassen sich durch Umsatz mit organischen oder anorganischen Säuren der Formel HB in ihre Säureadditionssalze der allgemeinen Formel I x HB überführen.Als anorganische Säuren HB kommen beispielsweise in Betracht: Halogenwasserstoffsäuren wie Chlorwasserstoffsäure und Bromwasserstoffsäure, sowie Schwefelsäure, Phosphorsäure und Amidosulfonsäure. Als organische Säuren HB seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Es wurden außer den in den Ausführungsbeispielen beschriebenen Derivaten auch die in den folgenden Tabellen zusammengestellten Verbindungen der allgemeinen Formel I bzw. deren Säureadditionsprodukte erhalten:

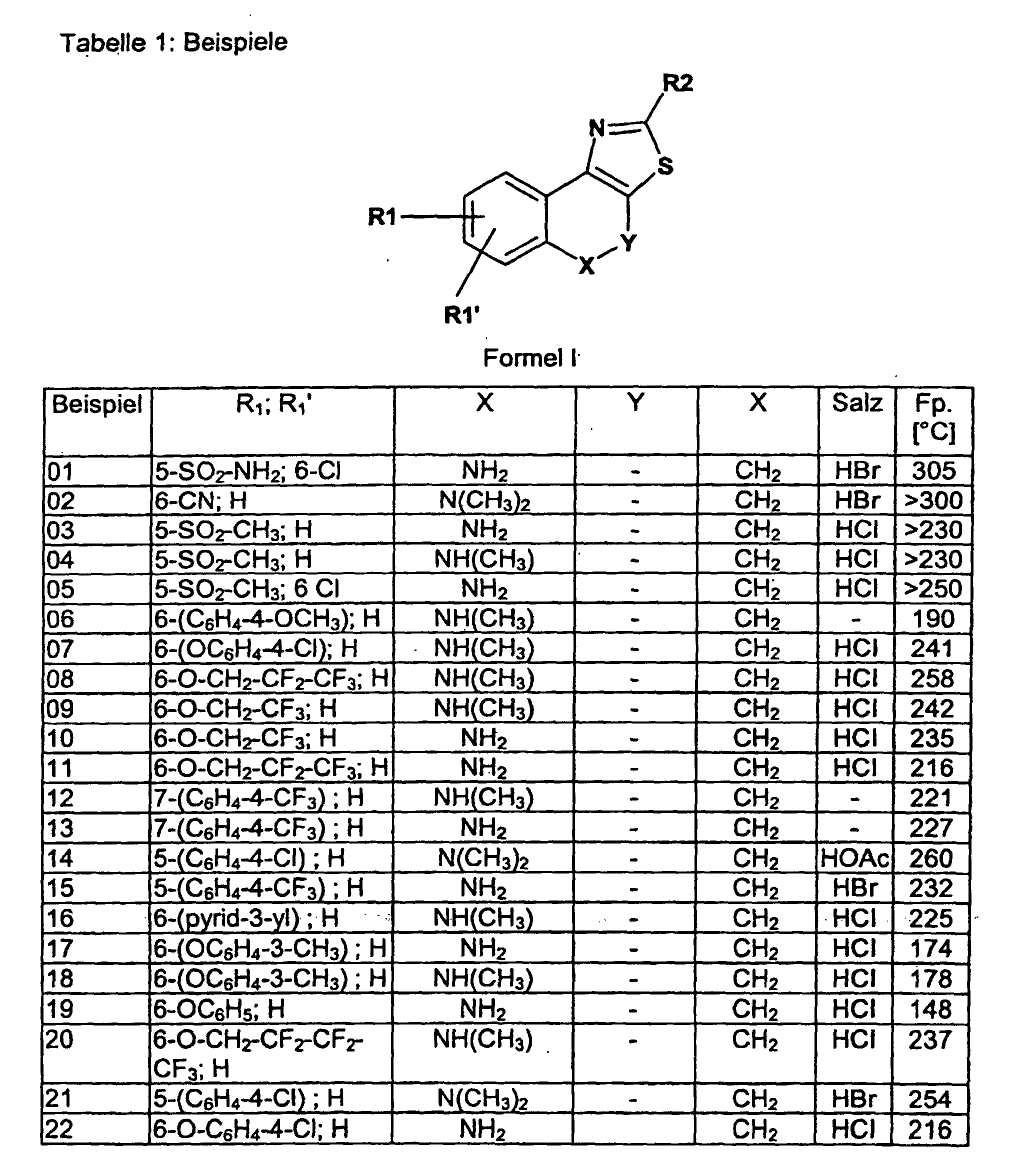

Die Verbindungen der Formel I zeichnen sich durch günstige Wirkungen auf den Fettstoffwechsel aus, insbesondere sind sie als Anorektika geeignet. Die Verbindungen können allein oder in Kombination mit weiteren anorektischen Wirkstoffen eingesetzt werden. Solche weiteren anorektischen Wirkstoffe werden z.B. in der Roten Liste, Kapitel 01 unter Abmagerungsmittel/Appetitzügler genannt. Die Verbindungen eignen sich zur Prophylaxe sowie insbesondere zur Behandlung von Obesitas. Die Verbindungen eignen sich weiterhin zur Prophylaxe sowie insbesondere zur Behandlung von Typ II Diabetes.

Die Wirksamkeit der Verbindungen wurde wie folgt getestet:

### Biologisches Prüfmodell:

Die Prüfung der anorektischen Wirkung erfolgte an männlichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

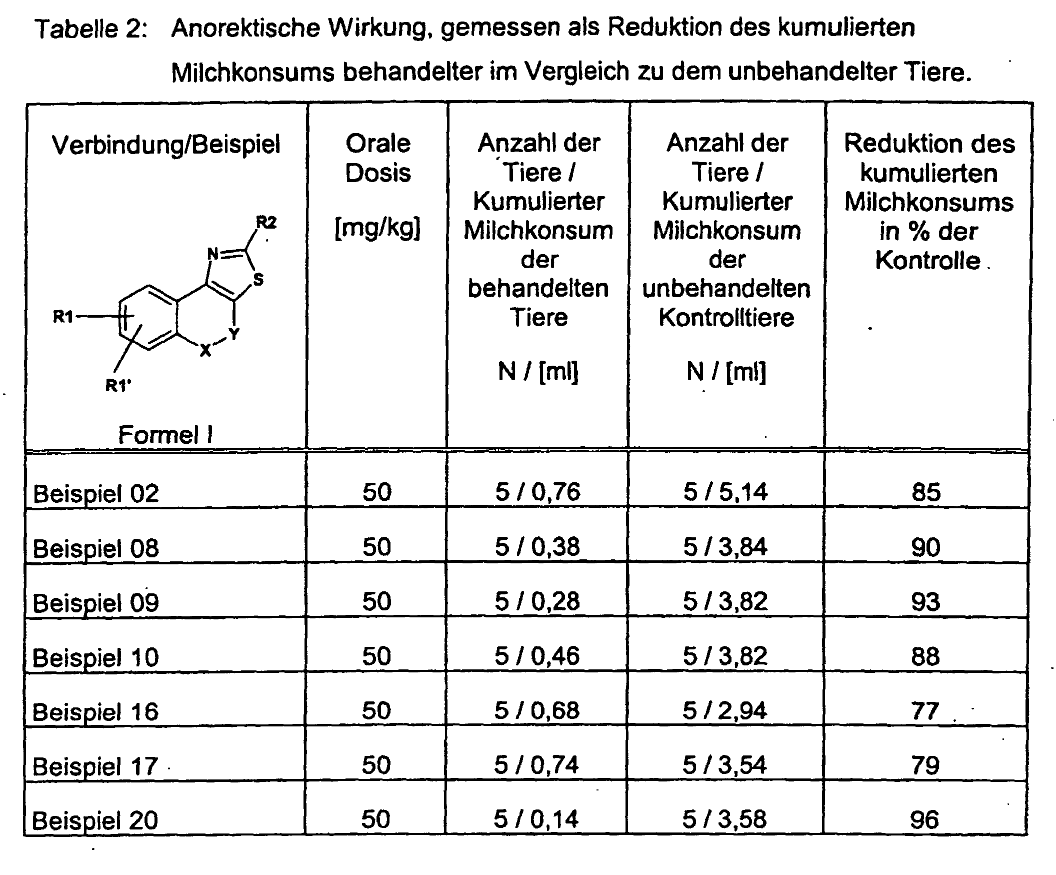

Die nachfolgend aufgeführten Beispiele dienen zur Erläuterung der Erfindung, ohne diese jedoch einzuschränken. Die angegebenen Zersetzungspunkte sind nicht korrigiert und generell von der Aufheizgeschwindigkeit abhängig.

### Ausführungsbeispiel 1:

### 2-Dimethylamino-8H-indeno[1,2-d]thiazol-6-carbonitril Hydrobromid (Verbindung des Beispiels 02):

a) 1-Oxo-indan-5-carbonitril:
   9,5 g 5-Brom-indan-1-on und 4,93 g CuCN werden in 10 ml Dimethylformamid suspendiert und 4 Stunden unter Rückfluß gekocht. Zur abgekühlten, dunkelbraunen, viskosen Suspension wird unter Rühren eine Lösung von 18 g Eisen-III-chlorid in 5 ml konz. Salzsäure mit 30 ml Wasser zugetropft und anschließend für 30 Minuten bei 70°C gerührt. Die Reaktionsmischung wird dreimal mit 50 ml Toluol ausgeschüttelt, und die vereinigten organischen Phasen werden mit 50 ml 2N Salzsäure und 50 ml 2 N Natronlauge ausgeschüttelt und dann mit Wasser neutral gewaschen. Der Toluolextrakt wird über Magnesiumsulfat getrocknet, im Vakuum eingeengt, und der Rückstand wird aus n-Heptan umkristallisiert. Man erhält 1-Oxo-indan-5-carbonitril mit dem Schmelzpunkt 123 - 125°C.
b) 2-Brom-1-oxo-indan-5-carbonitril:
   Die Bromierung des 1-Oxo-indan-5-carbonitrils erfolgt mit Brom in Eisessig unter Zufügung einer katalytischen Menge 48%iger HBr-Lösung in Wasser und liefert 2-Brom-1-oxo-indan-5-carbonitril mit dem Schmelzpunkt 115 - 118°C.
c) 2-Dimethylamino-8H-indeno[1,2-d]thiazol-6-carbonitril Hydrobromid:
   236 mg 2-Brom-1-oxo-indan-5-carbonitril werden mit 156 mg N,N-Dimethylthioharnstoff in 10 ml trockenem Aceton 3 h unter Rückfluß erhitzt. Die Reaktionsmischung wird im Vakuum eingeengt; der Rückstand mit wenig Aceton verrührt, abgesaugt, mit Aceton gewaschen und im Vakuum getrocknet. Man erhält 2-Dimethylamino-8H-indeno[1,2-d]thiazol-6-carbonitril Hydrobromid mit dem Schmelzpunkt > 300°C.

### Ausführungsbeispiel 2:

### 5-Methansulfonyl-8H-indeno[1,2-d]thiazol-2-yl-amin Hydrochlorid (Verbindung des Beispiels 03):

a) 2-Amino-5-methansulfonyl-8,8a-dihydro-indeno[1,2-d]thiazol-3a-ol Hydrochlorid:
   2.3 g 2-Brom-6-methansulfonyl-indan-1-on werden in 50 ml Aceton gelöst und unter Rühren mit 0.67 g Thioharnstoff versetzt. Aus der zunächst klaren Lösung kristallisiert nach einigen Minuten das Hydrobromid der ringeschlossenen Verbindung aus. Man rührt 4 h bei Raumtemp. nach, saugt ab, löst den Feststoff in ca. 30 ml Methanol und setzt 1 ml Triethylamin zu. Wiederum setzt nach wenigen Minuten Niederschlagsbildung ein. Nach 15 min gibt man 150 ml Wasser dazu und vervollständigt die Produktbildung durch Nachrühren bei Raumtemp. Der Niederschlag wird abgesaugt, mit Wasser nachgewaschen und an der Luft getrocknet. Durch Lösen in Essigester, Zusatz von etherischer Salzsäure, Absaugen des gebildeten Produkts und Trocknen i. Vak. erhält man das Hydrochlorid des 2-Amino-5-methansulfonyl-8,8a-dihydro-indeno[1,2-d]thiazol-3a-ols mit dem Zersetzungspunkt 241 °C.
b) 5-Methansulfonyl-8H-indeno[1,2-d]thiazol-2-yl-amin Hydrochlorid:
   1g der unter a) gewonnenen Verbindung wird in 100 ml halbconc. Salzsäure 10 h bei Raumtemp. gerührt, das Produkt abgesaugt und mit kaltem Wasser kurz nachgewaschen. Man erhält 5-Methansulfonyl-8Hindeno[1,2-d]thiazol-2-yl-amin Hydrochlorid vom Schmelzpunkt 230 °C.

### Ausführungsbeispiel 3:

### 6-Chlor-5-methansulfonyl-8H-indeno[1,2-d]thiazol-2-yl-amin Hydrochlorid (Verbindung des Beispiels 05):

Ausgehend von 2-Brom-5-chlor-6-methansulfonyl-indan-1-on gewinnt man in der Art und Weise wie vorgehend beschrieben 6-Chlor-5-methansulfonyl-8H-indeno[1,2-d]thiazol-2-yl-amin Hydrochlorid mit dem Schmelzpunkt >260°C.

### Ausführungsbeispiel 4:

### Methyl-[6-(2,2,3,3,3-pentafluor-propoxy)-8H-indeno[1,2-d]thiazol-2-yl]-amin Hydrochlorid (Verbindung des Beispiels 08):

a) 5-(2,2,3,3,3-pentafluor-propoxy)-indan-1-on:
   6.5 g 5-Fluor-indan-1-on werden in 50 ml trockenem Dimethylacetamid gelöst, mit 35.6 g wasserfreiem, gemahlenem Kaliumcarbonat und 12.9 g 2,2,3,3,3-Pentafluorpropanol versetzt und 10 h bei 95-100°C gerührt. Danach wird das Lösemittel im Vakuum abdestilliert; der Rückstand wird mit 300 ml Wasser versetzt, und die wässrige Phase wird mehrmals mit Essigsäureethylester extrahiert. Die organische Phase wird mit Wasser gewaschen, über Natriumsulfat getrocknet und im Vakuum eingeengt. Die Reinigung an Kieselgel liefert 5-(2,2,3,3,3-pentafluor-propoxy)-indan-1-on als ein braunes Öl, welches nach einiger Zeit kristallisiert; Schmelzpunkt 52-54°C.
b) 2-Brom-5-(2,2,3,3,3-pentafluor-propoxy)-indan-1-on:
   6.9 g 5-(2,2,3,3,3-pentafluor-propoxy)-indan-1-on werden in 100 ml Essigsäureethylester gelöst und tropfenweise mit einer Lösung von 3.9 g Brom in 15 ml Essigsäureethylester versetzt. Die Lösung wird kurz zum Rückfluß erhitzt, bevor der Rest der Bromlösung zugetropft wird. Anschließend wird 2 h bei Raumtemperatur gerührt. Die Reaktionslösung wird im Vakuum eingeengt und liefert 2-Brom-5-(2,2,3,3,3-pentafluorpropoxy)-indan-1-on als Öl, welches ohne weitere Reinigung in die nächste Stufe eingesetzt wird.
c) Methyl-[6-(2,2,3,3,3-pentafluor-propoxy)-8H-indeno[1,2-d]thiazol-2-yl]-amin Hydrochlorid:
   1.79 g 2-Brom-5-(2,2,3,3,3-pentafluor-propoxy)-indan-1-on werden in 60 ml Essigsäureethylester gelöst und mit einer Suspension aus 450 mg N-Methylthiohamstoff in 20 ml Essigsäureethylester versetzt. Die Reaktionslösung wird 7 h bei Raumtemperatur gerührt; der helle Niederschlag wird abgesaugt und mit Essigsäureethylester gewaschen und anschließend getrocknet. Das erhaltene Hydrobromid wird in 60 ml Methanol gelöst, mit 1.53 g Triethylamin versetzt und 5 h bei Raumtemperatur gerührt. Die Lösung wird eingeengt; der Rückstand kristallisiert bei Zugabe von Wasser. Die getrocknete freie Base wird in Essigsäureethylester gelöst und mit etherischer HCl-Lösung bis zur sauren Reaktion versetzt. Nach 3 h bei Raumtemperatur werden die gebildeten Kristalle abgesaugt und im Vakuum getrocknet. Zur Herstellung des ungesättigten Systems werden die getrockneten Kristalle in 35 ml Eisessig 2 h zum Rückfluß erhitzt. Das Lösemittel wird im Vakuum abdestilliert und der feste Rückstand wird mit Diisopropylether verrührt, abgesaugt und im Vakuum getrocknet. Man erhält Methyl-[6-(2,2,3,3,3-pentafluor-propoxy)-8H-indeno[1,2-d]thiazol-2-yl]-amin Hydrochlorid mit dem Schmelzpunkt 258°C.

### Ausführungsbeispiel 5:

### Methyl-(6-pyridin-3-yl-8H-indeno[1,2-d]thiazol-2-yl)-amin Hydrochlorid (Verbindung des Beispiels 16):

a) 5-Pyridin-3-yl-indan-1-on:
   13,26 g 3-Brompyridin werden in 160 ml Diethylether gelöst und auf -60°C abgekühlt. Zu dieser Lösung werden während 30 Minuten 52 ml einer 1,6 molaren Lösung von n-Butyllithium in n-Hexan zugetropft. Man läßt die Lösung sich auf -30°C erwärmen und fügt bei dieser Temperatur tropfenweise unter Rühren 9,5 ml Borsäuretrimethylester zu. Das Reaktionsgemisch wird anschließend 3 Stunden unter Rückfluß erhitzt, dann auf 0°C abgekühlt und tropfenweise mit 6,1 ml 1,3-Propandiol versetzt. Diese Mischung wird 30 Minuten bei 0°C gerührt, bevor 5,46 ml Methansulfonsäure zugetropft werden und weitere 30 Minuten gerührt wird. Danach fügt man 20 g Celite zu, erwärmt das Gemisch auf Raumtemperatur, filtriert, engt das Filtrat ein, verrührt den Rückstand in 700 ml Toluol, filtriert erneut und destilliert das Lösemittel im Vakuum ab. 4,1 g des Rückstands (3-[1,3,2]Dioxaborinan-2-yl-pyridin) werden ohne weitere Reinigung zusammen mit 4,22 g 5-Bromindan-1-on und 4,24 g Natriumcarbonat in einer Mischung aus 100 ml Toluol mit 20 ml Ethanol und 20 ml Wasser gelöst. Die Lösung wird mit Argon entgast, und anschließend werden 112 mg Palladium-II-acetat und 262 mg Triphenylphosphin zugegeben. Das Reaktionsgemisch wird 4 Stunden unter Rückfluß gekocht, auf Raumtemperatur abgekühlt und der Ethanolanteil des Gemisches wird im Vakuum abdestilliert. Sodann werden unter Rühren 50 ml einer 0,5 N Natronlauge zugesetzt, die organische Phase abgetrennt und die wässrige Phase mit Toluol ausgeschüttelt. Die vereinigten organischen Phasen werden nacheinander mit Wasser und ges. Kochsalzlösung ausgeschüttelt, über Magnesiumsulfat getrocknet, im Vakuum eingeengt und über Kieselgel mit Essigsäureethylester / n-Heptan 1/1 chromatographisch gereinigt. Man erhält 5-pyridin-3-yl-indan-1-on mit dem Schmelzpunkt 103 - 106°C.
b) 2-Chlor-5-pyridin-3-yl-indan-1-on:
   3,22 g 5-pyridin-3-yl-indan-1-on werden in 160 ml Dichlormethan gelöst und bei 0°C während 15 Minuten tropfenweise mit einer Lösung von 1,34 ml Sulfurylchlorid in 40 ml Dichlormethan versetzt. Es wird 30 Minuten bei 0°C und dann 60 Minuten bei Raumtemperatur nachgerührt, bevor man langsam 50 ml einer ges. Natriumhydrogencarbonatlösung zufügt. Die organische Phase wird abgetrennt, mit Wasser gewaschen, über Magnesiumsulfat getrocknet, im Vakuum eingeengt und über Kieselgel mit Dichlormethan / Methanol 50/1 chromatographisch gereinigt. Man erhält (neben 2,2-Dichlor-5-pyridin-3-yl-indan-1-on mit dem Schmelzpunkt 109°C) 2-Chlor-5-pyridin-3-yl-indan-1-on mit dem Schmelzpunkt 103-105°C.
c) Methyl-(6-pyridin-3-yl-8H-indeno[1,2-d]thiazol-2-yl)-amin Hydrochlorid:
   366 mg 2-Chlor-5-pyridin-3-yl-indan-1 -on werden mit 203 mg N-Methylthiohamstoff in 5 ml Methanol gelöst und 7 h zum Rückfluß erhitzt. Die abgekühlte Reaktionsmischung wird mit 20 ml Aceton versetzt, der Niederschlag wird abgesaugt, mit Aceton gewaschen und im Vakuum getrocknet. Man erhält Methyl-(6-pyridin-3-yl-8H-indeno[1,2-d]thiazol-2-yl)-amin Hydrochlorid mit dem Schmelzpunkt 225°C.

### Ausführungsbeispiel 6:

### 6-m-Tolyloxy-8H-indeno[1,2-d]thiazol-2-yl-amin Hydrochlorid (Verbindung des Beispiels 17):

a) 5-m-Tolyloxy-indan-1-on:
   5 g 5-Fluor-indan-1-on werden in 50 ml trockenem Dimethylformamid gelöst und mit 18.2 g wasserfreiem, gepulvertem Kaliumcarbonat und 3.57 g m-Kresol versetzt. Die Reaktionsmischung wird 6 h bei 110°C gerührt. Das Lösemittel wird im Vakuum abdestilliert und der Rückstand wird mit 100 ml Wasser versetzt und 2 h gerührt. Der wässrige Rückstand wird mit Essigsäureethylester extrahiert und der organische Extrakt wird 3 x mit Wasser gewaschen, über Natriumsulfat getrocknet, filtriert und im Vakuum eingeengt. Man erhält 5-m-Tolyloxy-indan-1-on als ein braunes Öl, welcher ohne weitere Reinigung weiter umgesetzt wird.
b) 2-Brom-5-m-tolyloxy-indan-1-on:
   Die Bromierung von 5-m-Tolyloxy-indan-1-on erfolgt analog der Bromierung von 5-(2,2,3,3,3-pentafluor-propoxy)-indan-1-on (Ausführungsbeispiel 4) und liefert 2-Brom-5-m-tolyloxy-indan-1-on als hellbraunes Öl.
c) 6-m-Tolyloxy-8H-indeno[1,2-d]thiazol-2-yl-amin Hydrochlorid:
   1.4 g des obigen Bromketons werden in 14 ml Aceton gelöst, mit 340 mg Thioharnstoff in 20 ml Aceton vesetzt und 7 h bei Raumtemperatur gerührt. Die ausgefallenen Kristalle (2-Amino-6-m-tolyloxy-8,8a-dihydro-indeno[1,2-d]thiazol-3a-ol Hydrobromid) werden abgesaugt und im Vakuum getrocknet. Wie im Ausführungsbeispiel 4 beschrieben, wird auch dieses Hydrobromid in die freie Base und weiter in das Hydrochlorid überführt. Das Hydrochlorid des 2-Amino-6-m-tolyloxy-8,8a-dihydro-indeno[1,2-d]thiazol-3a-ols wird in 30 ml Eisessig supendiert und unter Rühren zum Rückfluß erhitzt. Nach 2 h wird die Lösung im Vakuum eingeengt, der Rückstand mit Diisopropylether verrührt, abgesaugt und im Vakuum getrocknet. Man erhält 6-m-Tolyloxy-8H-indeno[1,2-d]thiazol-2-yl-amin Hydrochlorid mit dem Schmelzpunkt 174°C.

## Patentansprüche

1. Verwendung der Verbindungen der Formel I, 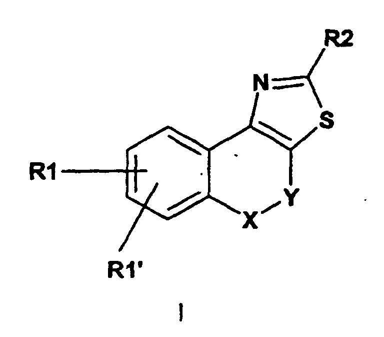 worin bedeuten
Y eine direkte Bindung;
X CH₂;
R1 CF₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkyl-, Alkenyl- und Alkinylresten ein, mehrere, oder alle Wasserstoff(e) durch Fluor ersetzt ist;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
Phenyl, O-Phenyl, wobei der Phenylrest bis zu 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R1' H, CF₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkyl-, Alkenyl- und Alkinylresten ein, mehrere, oder alle Wasserstoff(e) durch Fluor ersetzt ist;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
Phenyl, O-Phenyl, wobei der Phenylrest bis zu 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R2 NH₂, NHR3, NR4R5;
R3 (C₁-C₆)-Alkyl;
R4 (C₁-C₆)-Alkyl;
R5 (C₁-C₆)-Alkyl;
sowie derer physiologisch verträglichen Salze zur Herstellung eines Medikaments zur Behandlung von Fettstoffwechselstörungen.

2. Verwendung der Verbindungen der Formel I, 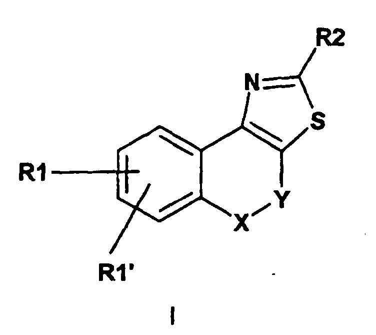 worin bedeuten
Y eine direkte Bindung;
X CH₂;
R1 CF₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkyl-, Alkenyl- und Alkinylresten ein, mehrere, oder alle Wasserstoff(e) durch Fluor ersetzt ist;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
Phenyl, O-Phenyl, wobei der Phenylrest bis zu 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R1' H, CF₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkyl-, Alkenyl- und Alkinylresten ein, mehrere, oder alle Wasserstoff(e) durch Fluor ersetzt ist;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
Phenyl, O-Phenyl, wobei der Phenylrest bis zu 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R2 NH₂, NHR3, NR4R5;
R3 (C₁-C₆)-Alkyl;
R4 (C₁-C₆)-Alkyl;
R5 (C₁-C₆)-Alkyl;
sowie derer physiologisch verträglichen Salze zur Herstellung eines Medikaments zur Behandlung von Obesitas.

3. Verwendung der Verbindungen der Formel I, 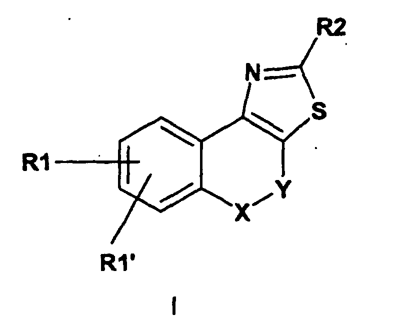 worin bedeuten
Y eine direkte Bindung;
X CH₂;
R1 CF₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkyl-, Alkenyl- und Alkinylresten ein, mehrere, oder alle Wasserstoff(e) durch Fluor ersetzt ist;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
Phenyl, O-Phenyl, wobei der Phenylrest bis zu 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R1' H, CF₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkyl-, Alkenyl- und Alkinylresten ein, mehrere, oder alle Wasserstoff(e) durch Fluor ersetzt ist;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
Phenyl, O-Phenyl, wobei der Phenylrest bis zu 3-fach substituiert sein kann mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R2 NH₂, NHR3, NR4R5;
R3 (C₁-C₆)-Alkyl;
R4 (C₁-C₆)-Alkyl;
R5 (C₁-C₆)-Alkyl;
sowie derer physiologisch verträglichen Salze zur Herstellung eines Medikaments zur Behandlung von Typ II Diabetes.

## Claims

1. The use of compounds of the formula I 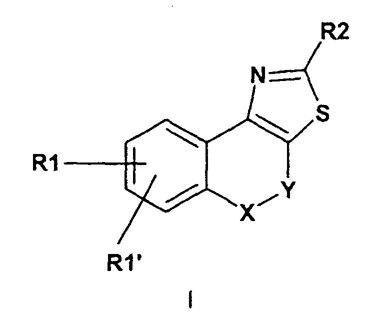 in which
Y is a direct linkage;
X is CH₂;
R1 is CF₃, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more than one or all hydrogen(s) in the alkyl, alkenyl and alkynyl radicals is replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to twice by F, Cl, Br, OH, CF₃, NO₂, CN, OCF₃, O-(C₁-C₆)-alkyl, (C₁-C₆)-alkyl, NH₂;
phenyl, O-phenyl, it being possible for the phenyl radical to be substituted up to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R1' is H, CF₃, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more than one or all hydrogen(s) in the alkyl, alkenyl and alkynyl radicals are replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to twice by F, Cl, Br, OH, CF₃, NO₂, CN, OCF₃, O-(C₁-C₆)-alkyl, (C₁-C₆)-alkyl, NH₂;
phenyl, O-phenyl, it being possible for the phenyl radical to be substituted up to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R2 is NH₂, NHR3, NR4R5;
R3 is (C₁-C₆)alkyl;
R4 is (C₁-C₆)-alkyl;
R5 is (C₁-C₆)-alkyl;
and its physiologically tolerated salts for the manufacture of a medicament for the treatment of disorders of lipid metabolism.

2. The use of compounds of the formula I 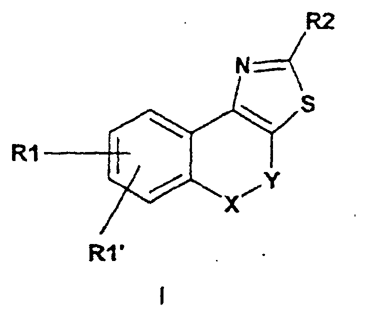 in which
Y is a direct linkage;
X is CH₂;
R1 is CF₃, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more than one or all hydrogen(s) in the alkyl, alkenyl and alkynyl radicals are replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to twice by F, Cl, Br, OH, CF₃, NO₂, CN, OCF₃, O-(C₁-C₆)-alkyl, (C₁-C₆)-alkyl, NH₂;
phenyl, O-phenyl, it being possible for the phenyl radical to be substituted up to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R1' is H, CF₃, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more than one or all hydrogen(s) in the alkyl, alkenyl and alkynyl radicals are replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to twice by F, Cl, Br, OH, CF₃, NO₂, CN, OCF₃, O-(C₁-C₆)-alkyl, (C₁-C₆)-alkyl, NH₂;
phenyl, -O-phenyl, it being possible for the phenyl radical to be substituted up to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R2 is NH₂, NHR3, NR4R5;
R3 is (C₁-C₆)alkyl;
R4 is (C₁-C₆)-alkyl;
R5 is (C₁-C₆)-alkyl;
and its physiologically tolerated salts for the manufacture of a medicament for the treatment of obesity.

3. The use of compounds of the formula I 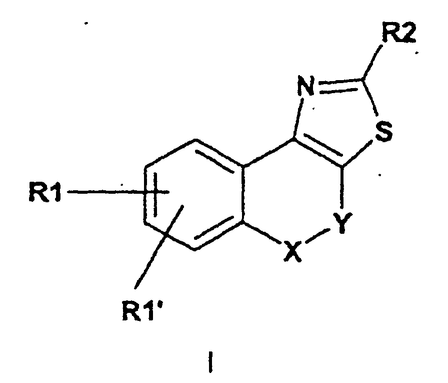 in which
Y is a direct linkage;
X is CH₂;
R1 is CF₃, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more than one or all hydrogen(s) in the alkyl, alkenyl and alkynyl radicals are replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to twice by F, Cl, Br, OH, CF₃, NO₂, CN, OCF₃, O-(C₁-C₆)-alkyl, (C₁-C₆)-alkyl, NH₂;
phenyl, O-phenyl, it being possible for the phenyl radical to be substituted up to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R1' is H, CF₃, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more than one or all hydrogen(s) in the alkyl, alkenyl and alkynyl radicals are replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to twice by F, Cl, Br, OH, CF₃, NO₂, CN, OCF₃, O-(C₁-C₆)-alkyl, (C₁-C₆)-alkyl, NH₂;
phenyl, O-phenyl, it being possible for the phenyl radical to be substituted up to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R2 is NH₂, NHR3, NR4R5;
R3 is (C₁-C₆)alkyl;
R4 is (C₁-C₆)-alkyl;
R5 is (C₁-C₆)-alkyl;
and its physiologically tolerated salts for the manufacture of a medicament for the treatment of type II diabetes.

## Revendications

1. Utilisation des composés de formule I, 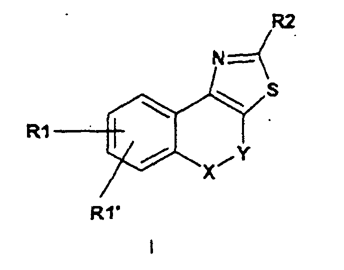 dans laquelle
Y représente une liaison directe ;
X représente CH₂ ;
R1 représente CF₃, CN, COOH, un groupe COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, O-alkyle en C₁-C₆, un, plusieurs ou tous les atome(s) d'hydrogène dans les radicaux alkyle, alcényle et alcynyle étant remplacé(s) par le fluor ;
SO₂-NH₂, SO₂NH-alkyle(C₁-C₆), SO₂N[alkyle-(C₁-C₆)]₂, S-alkyle(C₁-C₆), S- (CH₂)ₙ-phényle, SO-alkyle (C₁-C₆), SO-(CH₂)ₙ-phényle, SO₂-alkyle(C₁-C₆), SO₂-(CH₂)ₙ-phényle, n pouvant être 0-6 et le radical phényle pouvant être substitué jusqu'à deux fois par un ou des atomes de F, Cl, Br ou groupes OH, CF₃, NO₂, CN, OCF₃, O-alkyle en C₁-C₆, alkyle en C₁-C₆, NH₂ ;
phényle, O-phényle, le radical phényle pouvant être substitué jusqu'à 3 fois par un ou des atomes de F, Cl, Br, I ou groupes OH, CF₃, NO₂, CN, OCF₃, O-alkyle(C₁-C₆), alkyle en C₁-C₆, NH₂, NH-alkyle(C₁-C₆), N(alkyle(C₁-C₆))₂, SO₂-CH₃, COOH, COO-alkyle(C₁-C₆), CONH₂ ;
R1' représente H, CF₃, CN, COOH, un groupe COO-alkyle(C₁-C₆), CONH₂, CONH-alkyle (C₁-C₆), CON[alkyle(C₁-C₆)]₂, alkyle en C₁-C₆, alcényle en C₂-C₆, alcynyle en C₂-C₆, O-alkyle en C₁-C₆, un, plusieurs ou tous les atome(s) d'hydrogène dans les radicaux alkyle, alcényle et alcynyle étant remplacé(s) par le fluor ;
SO₂-NH₂, SO₂NH-alkyle(C₁-C₆), SO₂N[alkyle-(C₁-C₆)]₂, S-alkyle (C₁-C₆), S-(CH₂)ₙ-phényle, SO-alkyle(C₁-C₆), SO- (CH₂)ₙ-phényle, SO₂-alkyle(C₁-C₆), SO₂-(CH₂)ₙ-phényle, n pouvant être 0-6 et le radical phényle pouvant être substitué jusqu'à deux fois par un ou des atomes de F, Cl, Br ou groupes OH, CF₃, NO₂, CN, OCF₃, O-alkyle en C₁-C₆, alkyle en C₁-C₆, NH₂ ;
phényle, O-phényle, le radical phényle pouvant être substitué jusqu'à 3 fois par un ou des atomes de F, Cl, Br, I ou groupes OH, CF₃, NO₂, CN, OCF₃, O-alkyle en C₁-C₆, alkyle en C₁-C₆, NH₂, NH-alkyle (C₁-C₆), N(alkyle(C₁-C₆))₂, SO₂-CH₃, COOH, COO-alkyle(C₁-C₆), CONH₂ ;
R2 représente NH₂, NHR3, NR4R5 ;
R3 représente un groupe alkyle en C₁-C₆:
R4 représente un groupe alkyle en C₁-C₆ ;
R5 représente un groupe alkyle en C₁-C₆ ;
ainsi que de leurs sels physiologiquement acceptables, pour la fabrication d'un médicament destiné au traitement de troubles lipométaboliques.

2. Utilisation des composés de formule I, 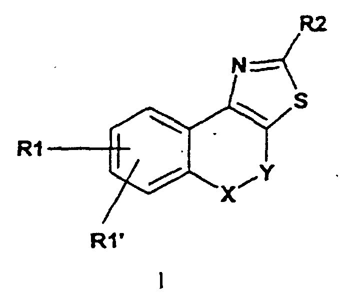 dans laquelle
Y représente une liaison directe ;
X représente CH₂ ;
R1 représente CF₃, CN, COOH, un groupe COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, O-alkyle en C₁-C₆, un, plusieurs ou tous les atome(s) d'hydrogène dans les radicaux alkyle, alcényle et alcynyle étant remplacé(s) par le fluor ;
SO₂-NH₂, SO₂NH-alkyle(C₁-C₆), SO₂N[alkyle-(C₁-C₆)]₂, S-alkyle(C₁-C₆), S- (CH₂)ₙ-phényle, SO-alkyle(C₁-C₆), SO-(CH₂)ₙ-phényle, SO₂-alkyle(C₁-C₆), SO₂-(CH₂)ₙ-phényle, n pouvant être 0-6 et le radical phényle pouvant être substitué jusqu'à deux fois par un ou des atomes de F, Cl, Br ou groupes OH, CF₃, NO₂, CN, OCF₃, O-alkyle en C₁-C₆, alkyle en C₁-C₆, NH₂ ;
phényle, O-phényle, le radical phényle pouvant être substitué jusqu'à 3 fois par un ou des atomes de F, Cl, Br, I ou groupes OH, CF₃, NO₂, CN, OCF₃, O-alkyle(C₁-C₆), alkyle en C₁-C₆, NH₂, NH-alkyle(C₁-C₆), N(alkyle(C₁-C₆))₂, SO₂-CH₃, COOH, COO-alkyle(C₁-C₆), CONH₂ ;
R1' représente H, CF₃, CN, COOH, un groupe COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, O-alkyle en C₁-C₆, un, plusieurs ou tous les atome(s) d'hydrogène dans les radicaux alkyle, alcényle et alcynyle étant remplacé(s) par le fluor ;
SO₂-NH₂, SO₂NH-alkyle(C₁-C₆), SO₂N[alkyle-(C₁-C₆)]₂, S-alkyle(C₁-C₆), S-(CH₂)ₙ-phényle, SO-alkyle(C₁-C₆), SO-(CH₂)ₙ-phényle, SO₂-alkyle(C₁-C₆), SO₂-(CH₂)ₙ-phényle, n pouvant être .0-6 et le radical phényle pouvant être substitué jusqu'à deux fois par un ou des atomes de F, Cl, Br ou groupes OH, CF₃, NO₂, CN, OCF₃, O-alkyle en C₁-C₆, alkyle en C₁-C₆, NH₂ ;
phényle, O-phényle, le radical phényle pouvant être substitué jusqu'à 3 fois par un ou des atomes de F, Cl, Br, I ou groupes OH, CF₃, NO₂, CN, OCF₃, O-alkyle en C₁-C₆, alkyle en C₁-C₆, NH₂, NH-alkyle(C₁-C₆), N(alkyle(C₁-C₆))₂, SO₂-CH₃, COOH, COO-alkyle(C₁-C₆), CONH₂ ;
R2 représente NH₂, NHR3, NR4R5 ;
R3 représente un groupe alkyle en C₁-C₆ ;
R4 représente un groupe alkyle en C₁-C₆ ;
R5 représente un groupe alkyle en C₁-C₆ ;
ainsi que de leurs sels physiologiquement acceptables, pour la fabrication d'un médicament destiné au traitement de l'obésité.

3. Utilisation des composés de formule I, 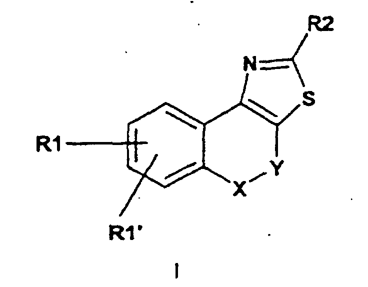 dans laquelle
Y représente une liaison directe ;
X représente, CH₂ ;
R1 représente CF₃, CN, COOH, un groupe COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, O-alkyle en C₁-C₆, un, plusieurs ou tous les atome(s) d'hydrogène dans les radicaux alkyle, alcényle et alcynyle étant remplacé(s) par le fluor ;
SO₂-NH₂, SO₂NH-alkyle(C₁-C₆), SO₂N[alkyle-(C₁-C₆)]₂, S-alkyle(C₁-C₆), S-(CH₂)ₙ-phényle, SO-alkyle(C₁-C₆), SO-(CH₂)ₙ-phényle, SO₂-alkyle(C₁-C₆), SO₂-(CH₂)ₙ-phényle, n pouvant être 0-6 et le radical phényle pouvant être substitué jusqu'à deux fois par un ou des atomes de F, Cl, Br ou groupes OH, CF₃, NO₂, CN, OCF₃, O-alkyle en C₁-C₆, alkyle en C₁-C₆, NH₂ ;
phényle, O-phényle, le radical phényle pouvant être substitué jusqu'à 3 fois par un ou des atomes de F, Cl, Br, I ou groupes OH, CF₃, NO₂, CN, OCF₃, O-alkyle(C₁-C₆), alkyle en C₁-C₆, NH₂, NH-alkyle(C₁-C₆), N(alkyle(C₁-C₆))₂, SO₂-CH₃, COOH, COO-alkyle(C₁-C₆), CONH₂ ;
R1' représente H, CF₃, CN, COOH, un groupe COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, O-alkyle en C₁-C₆, un, plusieurs ou tous les atome(s) d'hydrogène dans les radicaux alkyle, alcényle et alcynyle étant remplacé(s) par le fluor ;
SO₂-NH₂, SO₂NH-alkyle(C₁-C₆), SO₂N[alkyle-(C₁-C₆)]₂, S-alkyle (C₁-C₆), S-(CH₂)ₙ-phényle, SO-alkyle(C₁-C₆), SO- (CH₂)ₙ-phényle, SO₂-alkyle(C₁-C₆), SO₂-(CH₂)ₙ-phényle, n pouvant être 0-6 et le radical phényle pouvant être substitué jusqu'à deux fois par un ou des atomes de F, Cl, Br ou groupes OH, CF₃, NO₂, CN, OCF₃, O-alkyle en C₁-C₆, alkyle en C₁-C₆, NH₂ ;
phényle, O-phényle, le radical phényle pouvant être substitué jusqu'à 3 fois par un ou des atomes de F, Cl, Br, I ou groupes OH, CF₃, NO₂, CN, OCF₃, O-alkyle en C₁-C₆, alkyle en C₁-C₆, NH₂, NH-alkyle(C₁-C₆), N(alkyle(C₁-C₆))₂, SO₂-CH₃, COOH, COO-alkyle(C₁-C₆), CONH₂ ;
R2 représente NH₂, NHR3, NR4R5 ;
R3 représente un groupe alkyle en C₁-C₆ ;
R4 représente un groupe alkyle en C₁-C₆ ;
R5 représente un groupe alkyle en C₁-C₆ ;
ainsi que de leurs sels physiologiquement acceptables, pour la fabrication d'un médicament destiné au traitement du diabète de type II.
